# EUROPEAN PATENT APPLICATION

(11) **EP 4 607 192 A1**
(43) Date of publication of application: **27.08.2025**
(21) Application number: 23879784.9
(22) Date of filing: 17.10.2023
(51) Int. Cl.: G01N 33/53, C12M 1/00, C12M 1/34, C12Q 1/42, G01N 35/02, G01N 37/00

(54) **FLUID DEVICE, AND METHOD FOR DETECTING TARGET MOLECULE**

(30) Priority: 20.10.2022 JP 2022168340; 01.11.2022 JP 2022175384
(71) Applicant: Toppan Holdings Inc., Tokyo 110-0016 (JP)
(72) Inventor: HORIUCHI Yosuke, Tokyo 110-0016 (JP)
(74) Representative: Plasseraud IP
(86) International application number: PCT/JP2023/037512
(87) International publication number: WO 2024/085141

(57) **Abstract**

This fluidic device includes a substrate and a wall member provided on the substrate. The fluidic device further includes a plurality of first wells surrounded by the substrate and the wall member, and a plurality of second wells provided in the upper surface of the substrate corresponding to the bottoms of the first wells.

## Description

### [Technical Field]

The present invention relates to a fluidic device and a method for detecting a target molecule.

This application is based on and claims the benefit of priorities from earlier Japanese Patent Application No. 2022-168340 filed October, 20, 2022 and earlier Japanese Patent Application No. 2022-175384 filed November, 1, 2022, the descriptions of which are incorporated herein by reference.

### [Background Art]

Early detection of disease and prediction of the effects of medication are performed by quantitatively detecting a target molecule in a biological sample. For example, when the target molecule is a protein, quantification is performed by enzyme-linked immunosorbent assay (ELISA) or the like. When the target molecule is a nucleic acid, quantification is performed by real-time PCR or the like.

In recent years, there is an increasing need for detecting target molecules such as those described above more accurately for the purpose of, for example, finding disease earlier. For example, PTL 1, PTL 2, and NPL 1 disclose single-molecule detection techniques in which an enzymatic reaction is performed in a large number of micro compartments as techniques for accurately detecting a target molecule. These techniques are called digital measurement.

In digital measurement, a sample solution is filled into a reaction vessel (fluidic device) with an extremely large number of micro compartments so that the sample solution is distributed into the micro compartments. The number of target molecules is counted by binarizing the signal from each micro compartment and only determining (detecting) whether the target molecule contained in the sample solution is present in the micro compartment. Digital measurements can significantly improve detection sensitivity and quantifiability compared to ELISAs, real-time PCRs, etc. of the conventional art.

### [Citation List]

### [Patent Literature]

[PTL 1] JP 5551798 B
[PTL 2] JP 2 0 1 4 - 5 0 3 8 3 1 T

### [Non Patent Literature]

[NPL 1] Kim S. H., et al., Large-scale femtoliter droplet array for digital counting of single biomolecules., Lab on a Chip, 12 (23), 4986-4991, 2012.

### [Summary of the Invention]

### [Technical Problem]

In digital measurement techniques, target molecules in the solution sample are distributed into micro compartments so that a single molecule is contained in a micro compartment, and the number of micro compartments to which the target molecules have been distributed is counted. However, as the need for more accurate detection of target molecules is increasing as described above, there is a demand to go back to the source (that is, the target substance) of the target molecules and detect and quantify the amount of target molecules released from the target substance. For example, while conventional digital measurement techniques can detect cells (that is, the target substance) contained in a solution sample with high accuracy, they have trouble detecting and quantifying cytokines (that is, target molecules) secreted by cells with high accuracy. Further, there is a demand for digital measurements used in fields such as disease diagnosis and drug discovery to detect targets with higher accuracy.

The present invention has been made in view of the above circumstances, and an object thereof is to provide a fluidic device capable of detecting target molecules in a solution with high sensitivity. Another object thereof is to provide a method for detecting target molecules using such a fluidic device.

### [Solution to Problem]

In order to solve the above problems, one mode of the present invention encompasses the following modes.

[1-1] A fluidic device including: a substrate; a wall member provided on the substrate; and a cover member facing the substrate and in contact with the wall member, in which the cover member has an inlet and an outlet that pass through the cover member in a thickness direction, a flow channel through which a fluid flows is formed between a top of the wall member and the cover member, the fluidic device comprises a plurality of first wells surrounded by the substrate and the wall member, and the fluidic device comprises a plurality of second wells provided in an upper surface of the substrate corresponding to bottoms of the first wells.
[1-2] The fluidic device according to [1-1], in which the cover member is removable from the wall member.
[1-3] The fluidic device according to [1-1] or [1-2], in which an opening of the first wells has a major diameter of 1 µm or more and 1 cm or less.
[1-4] The fluidic device according to any one of [1-1] to [1-3], in which the first wells have an aspect ratio of 0.002 or more and 3 or less.
[1-5] The fluidic device according to any one of [1-1] to [1-4], in which an opening of the second wells has a major diameter of 1 nm or more and 1 mm or less.
[1-6] The fluidic device according to any one of [1-1] to [1-5], in which the second wells have an aspect ratio of 0.05 or more and 3 or less.
[1-7] A method for detecting a target molecule using the fluidic device according to any one of [1-1] to [1-6], the method including: filling the first wells independently with a sample solution containing a target substance; releasing the target molecule from the target substance in, among the first wells, first wells in which one unit of the target substance is placed; distributing the target molecule to the second wells independently so that one molecule is placed in one second well; and causing a reaction between the target molecule and a detection reagent to detect the target molecule.
[1-8] The method for detecting a target molecule according to [1-7], in which, in the filling step, a sealing liquid is introduced into the fluidic device to seal the sample solution in the first wells by the sealing liquid.
[1-9] The method for detecting a target molecule according to [1-8], in which the sealing liquid is fluorinated oil.
[1-10] The method for detecting a target molecule according to any one of [1-7] to [1-9], in which the sample solution contains a surfactant.
[1-11] The method for detecting a target molecule according to any one of [1-7] to [1-10], the method further including, prior to the filling step, obtaining the sample solution by adjusting a concentration so that one unit of the target substance is placed per first well based on a volume of the fluidic device.

As another aspect, one mode of the present invention encompasses the following modes.

[2-1] A method for detecting a target molecule using a fluidic device having wells, the method including: placing a reaction solution in the well for reaction between the target molecule, alkaline phosphatase immobilized on a binder that binds to the target molecule, and a substrate that changes visually when dephosphorylated by the alkaline phosphatase; and
   visualizing and detecting a presence of the target molecule contained in the wells by using a dephosphorylation reaction of the substrate by the alkaline phosphatase in the wells,
   in which, at a start of the step of visualizing and detecting the presence of the target molecule, a concentration of the substrate in the reaction solution contained in the wells is 80 µmol/L or more and 800 µmol/L or less.
[2-2] The method for detecting a target molecule according to [2-1], in which the reaction solution contains a surfactant.
[2-3] The method for detecting a target molecule according to [2-1] or [2-2], further including sealing the wells containing the reaction solution with oil.
[2-4] The method for detecting a target molecule according to any one of [2-1] to [2-3], in which the binder is an antibody.
[2-5] The method for detecting a target molecule according to any one of [2-1] to [2-4], in which, in the step of placing the target molecule in the wells, the target molecule is retained in the wells using particles to which a capture means for capturing the target molecule is immobilized.
[2-6] The method for detecting a target molecule according to any one of [2-1] to [2-5], in which the reaction solution contains a buffer solution.

### [Advantageous Effects of the Invention]

According to the present invention, a fluidic device capable detecting target molecules in a solution with high sensitivity can be provided. A method for detecting target molecules using such fluidic device can also be provided.

### [Brief Description of the Drawings]

[Fig. 1] Fig. 1 is a schematic perspective view illustrating a fluidic device according to a first embodiment.
[Fig. 2] Fig. 2 is a cross-sectional view taken along the line II-II of Fig. 1.
[Fig. 3] Fig. 3 is a diagram for explaining a method for detecting target molecules according to the first embodiment.
[Fig. 4] Fig. 4 is a diagram for explaining a method for detecting target molecules according to the first embodiment.
[Fig. 5] Fig. 5 is a diagram for explaining a method for detecting target molecules according to the first embodiment.
[Fig. 6] Fig. 6 is a diagram for explaining a method for detecting target molecules according to the first embodiment.
[Fig. 7] Fig. 7 is a diagram for explaining a method for detecting target molecules according to the first embodiment.
[Fig. 8] Fig. 8 is a schematic perspective view illustrating a fluidic device according to a second embodiment.
[Fig. 9] Fig. 9 is a cross-sectional view taken along the line IX-IX of Fig. 8.
[Fig. 10] Fig. 10 is a diagram for explaining a method for detecting target molecules according to the second embodiment.
[Fig. 11] Fig. 11 is a diagram for explaining a method for detecting target molecules according to the second embodiment.
[Fig. 12] Fig. 12 is a schematic cross-sectional view of a fluidic device according to another preferred embodiment of the present invention in a state where a liquid is being supplied thereto.
[Fig. 13] Fig. 13 is a schematic cross-sectional view of the fluidic device shown in Fig. 12 in a state where a sealing liquid is being supplied thereto.
[Fig. 14] Fig. 14 is a schematic cross-sectional view of the fluidic device shown in Fig. 12 in a state where the sealing liquid has been supplied thereto.
[Fig. 15] Fig. 15 is a schematic cross-sectional view of a fluidic device according to another embodiment of the present invention.
[Fig. 16] Fig. 16 is a bar graph showing the number of wells in which false positive reactions were observed for each substrate concentration.

### [Description of the Embodiments]

### [First Embodiment]

With reference to the drawings where necessary, a first embodiment of the present invention will be described in detail. Throughout the drawings, the same or corresponding parts are denoted by the same or corresponding reference signs to omit repeated explanation. The dimension ratios in some drawings are exaggerated for convenience of explanation and are not necessarily to scale.

### [Fluidic Device]

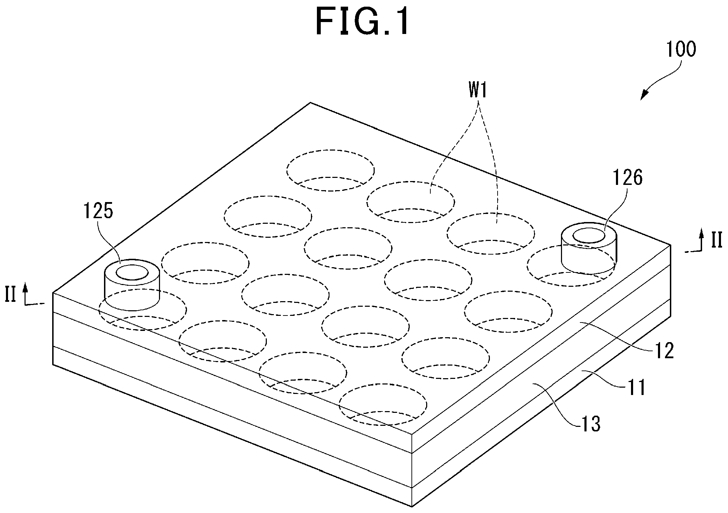

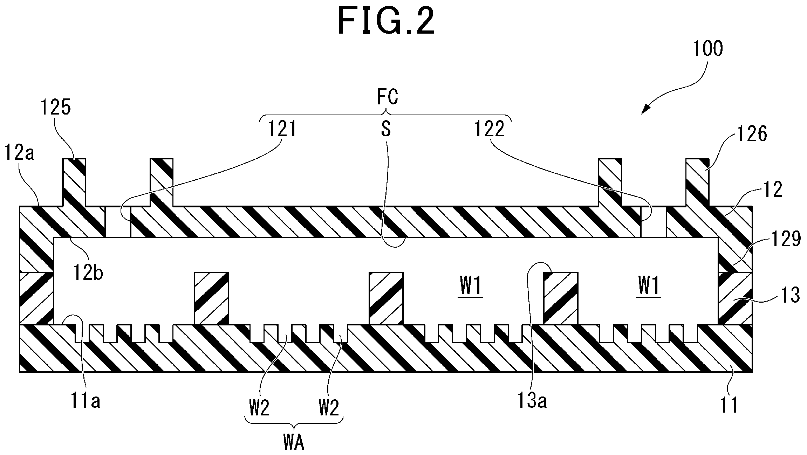

Fig. 1 is a schematic perspective view illustrating a fluidic device according to the present embodiment. Fig. 2 is a cross-sectional view taken along the line II-II of Fig. 1.

A fluidic device 100 includes a well plate (also called a substrate) 11, a cover member 12, and a wall member 13. The fluidic device 100 is used as a reaction vessel configured to contain a sample in an internal space S. In the reaction vessel, target molecules are released from a target substance in the sample, and a reaction for detecting the target molecules is induced.

The fluidic device 100 has a plurality of first wells W1 surrounded by the well plate 11 and the wall member 13, and a plurality of second wells W2 provided in an upper surface 11a of the well plate 11 corresponding to the bottoms of the first wells W1.

The following describes these components sequentially.

### (Well Plate)

The well plate 11 is a plate-like member having a rectangular shape in plan view. The term "plan view" refers to a view from the normal direction of the upper surface 11a of the well plate. The upper surface 11a of the well plate 11 is provided with a plurality of wells (also called second wells or microwells) W2.

The second wells W2 are recesses provided in the upper surface 11a of the well plate 11 and exposed to the internal space S the upper surface 11a. The term "second well W2" refers to the space surrounded by one of the recesses and an imaginary plane that is parallel to and in contact with the upper surface 11a.

The second wells W2 contain the sample contained in the internal space S and functions as a reaction field between the target molecule in the sample and a detection reagent.

The material of the well plate 11 may have or may not have electromagnetic wave transmission properties. Examples of electromagnetic waves that determine whether the material has transmission properties or not include an X-ray, ultraviolet ray, visible light, and infrared ray. When the well plate 11 has electromagnetic wave transmission properties, electromagnetic waves can be used to analyze the results of experiments performed with the fluidic device 100 having the well plate 11. For example, fluorescence, phosphorescence, or the like emitted as a result of applying electromagnetic waves can be measured from the well plate 11 side. The term "electromagnetic wave transmission properties" refers to properties that allow electromagnetic waves of various wavelengths to transmit, including radio waves, light, X-rays, and gamma rays.

The material having electromagnetic wave transmission properties may be, for example, glass, a resin, or the like. Examples of such resins include ABS resin, polycarbonate, cycloolefin copolymer (COC), cycloolefin polymer (COP), acrylic resin, polyvinyl chloride, polystyrene, polyethylene, polypropylene, polyvinyl acetate, polyethylene terephthalate (PET), polyethylene naphthalate (PEN), polycarbonate (PC), silicone resin, fluororesin, and amorphous fluororesin. These resins may contain various additives, or a plurality of resins may be mixed to form a polymer alloy.

It is preferred that the material with electromagnetic wave transmission properties practically does not exhibit autofluorescence. The phrase "practically does not exhibit autofluorescence" means that the material does not exhibit autofluorescence at all at any wavelength used for sample detection, or exhibits autofluorescence that is insignificantly weak and does not affect sample detection. For example, autofluorescence which is not more than one-half or not more than one-tenth of the fluorescence of the detection target can be regarded as being insignificant for the sample detection. When the well plate 11 is formed using such a material, the detection sensitivity can be increased when the sample is detected using electromagnetic waves.

For example, the material having electromagnetic wave transmission properties and which does not emit autofluorescence may be quartz glass. The material that emits insignificantly weak autofluorescence and does not hinder the sample detection using electromagnetic waves may be low fluorescence glass, an acrylic resin, a cycloolefin copolymer (COC), a cycloolefin polymer (COP), or the like.

The thickness of the well plate 11 can be determined as appropriate. If fluorescent light is to be observed from the well plate 11 side using a fluorescence microscope, the thickness of the well plate 11 may be, for example, 5 mm or less, 2 mm or less, or 1.6 mm or less.

The well plate 11 may be a single layer formed using only the above-described material, or a laminate of a plurality of materials. When the well plate 11 is formed by processing a laminate, the layer having the second wells W2 and the layer supporting this layer may be made of different materials. For example, a laminate of a substrate and a fluororesin layer stacked thereon may be used as the material of the well plate 11. The well array may be formed by processing the fluororesin layer. The fluororesin used may be, for example, CYTOP (trademark) (manufactured by AGC Inc.) or the like.

### (Microwells)

The second wells W2 can have various shapes. Examples of the shape of the second wells W2 include cylindrical shapes such as a cylinder, an elliptical cylinder, and a polygonal cylinder; shapes with a single apex such as a cone and a pyramid; and frustum shapes such as a truncated cone and a truncated pyramid. When the second wells W2 have a shape with a single apex or a frustum shape, it is preferable that the opening diameter gradually decreases towards the bottom of each well.

The bottom of the second wells W2 may be flat or curved (a convex or concave surface).

Each second well W2 may have a volume of approximately 10 fL to 1000 pL. In the fluidic device 100, the multiple second wells W2 with the same shape and size configure a well array WA. The phrase "same shape and size" means that the wells have shapes and volumes similar enough to perform digital measurement using the fluidic device 100, accepting a manufacturing error variation.

When the second wells W2 have a circular shape in plan view, the major diameter of the opening of each second well W2 may be, for example, 1 nm or more and 1 mm or less. The phrase "major diameter of each second well W2" refers to the length of the longer side of the smallest rectangle that circumscribes the opening of the second well W2 when the opening is viewed in plan view.

The aspect ratio of the second wells W2 is preferably 0.05 or more and 3 or less. The phrase "aspect ratio of the second wells W2" refers to the ratio of the depth of the second wells W2 to the major diameter of the second wells W2 ([depth of second wells W2] / [major diameter of second wells W2]).

The phrase "depth of the second wells W2" refers to the distance from the above-mentioned "imaginary plane that is parallel to and in contact with the upper surface 11a" to the bottom of each second well W2.

The arrangement of the second wells W2 is not particularly limited. The second wells W2 may be arranged, for example, in a triangular lattice shape or a square lattice shape, or may be irregularly arranged.

The well plate 11 can be manufactured by using a known injection molding method, microimprinting technique, or nanoimprinting technique. The well plate 11 can also be manufactured by forming the second wells W2 by etching using a known photolithography technique.

### (Wall Member)

The wall member 13 has a closed ring shape in plan view, and is placed on the upper surface 11a of the well plate 11.

The wall member 13 is sandwiched between the well plate 11 and the cover member 12 to be integrated with them and form the fluidic device 100. The space surrounded by the well plate 11, the cover member 12, and the wall member 13 is the internal space S in which a liquid sample is contained.

The wall member 13 serves as a wall surface of the internal space S, and also serves as a spacer between the well plate 11 and the cover member 12.

The internal space S is partitioned by the wall member 13 so that the first wells W1 are formed. The "first wells W1" each refer to a space surrounded by the well plate 11, the wall member 13, and an imaginary plane that is parallel to and in contact with a top 13a of the wall member 13.

The fluidic device 100 has 10 to 10,000 first wells W1. Further, although the area of the bottom surface of the first wells W1 is not particularly limited, it is preferably large enough to accommodate 10 to 10,000 of the above-described second wells W2. The major diameter of the opening of each first well W1 is preferably 1 µm or more and 1 cm or less. The phrase "major diameter of each first well W1" refers to the length of the longer side of the smallest rectangle that circumscribes the opening of the first well W1 when the opening is viewed in plan view.

The aspect ratio of the first wells W1 is preferably 0.002 or more and 3 or less. The phrase "aspect ratio of the first wells W1" refers to the ratio of the depth of the first wells W1 to the major diameter of the first wells W1 ([depth of first wells W1] / [major diameter of first wells W1]).

The phrase "depth of the first wells W1" refers to the distance from the above-mentioned "imaginary plane that is parallel to and in contact with a top 13a of the wall member 13" to the "imaginary plane that is parallel to and in contact with the upper surface 11a".

Each first well W1 may have a volume of approximately 100 fL to 1 µL.

The material of the wall member 13 may the same as that of the well plate 11 described above. The wall member 13 made of such a material can be integrated with the well plate 11 and the cover member 12 by bonding with an adhesive; or by welding such as heat welding, ultrasonic welding, or laser welding.

Note that the wall member 13 may be integrally formed with the well plate 11 and form a part of the well plate 11. Similarly, the wall member 13 may be integrally formed with the cover member 12 and form a part of the cover member 12.

### (Cover Member)

The cover member 12 has the same contour as the well plate 11 when viewed in plan view. The cover member 12 faces the well plate 11 and is in contact with the wall member 13.

The cover member 12 includes two through holes passing therethrough in the thickness direction. The two through holes are located at opposite ends of the cover member 12. One of the through holes is an inlet 121 used to inject a liquid into the internal space S of the fluidic device 100, and the other through hole is an outlet 122 used to discharge the liquid from the internal space S.

The term "liquid" not only refers to a liquid sample but also a detection reagent and sealing liquid.

The inlet 121, the internal space S, and the outlet 122 are connected in this order to form a flow channel FC as a whole. The fluidic device 100 allows a liquid to flow through the flow channel FC to cause a target substance detection reaction. More than one second well W2 is located between the inlet 121 and the outlet 122 when viewed in plan view.

The upper surface 12a of the cover member 12 is provided with a cylindrical injection port 125 surrounding the inlet 121. The injection port 125 is connected with the inlet 121. For example, the injection port 125 is used to connect a syringe filled with a liquid to fill the internal space with the liquid using the syringe.

Similarly, the upper surface 12a of the cover member 12 is provided with a cylindrical discharge port 126 surrounding the outlet 122. The discharge port 126 is connected with the outlet 122. The discharge port 126 is used, for example, to connect a tube through which the liquid flows when the liquid is removed from the internal space S.

A protruded part 129 is provided on the outer edge of a lower surface 12b of the cover member 12. The cover member 12 is connected to the outer edge of the wall member 13 via the protruded part 129.

The example materials for the well plate 11 described above can be used to form the cover member 12. The material of the cover member 12 may or may not be the same as the material of the well plate 11.

The material of the cover member 12 may or may not have electromagnetic wave transmission properties.

The cover member 12 can be manufactured by a known injection molding method.

Note that the fluidic device 100 may not have the cover member 12.

### <Method for Detecting Target Molecule>

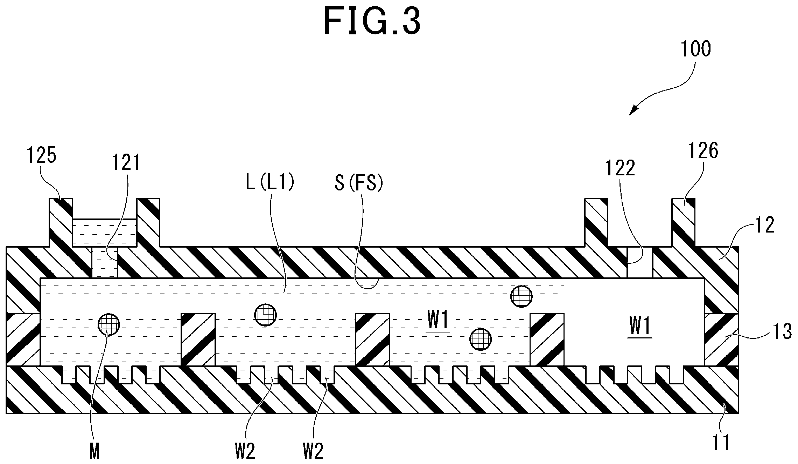

Figs. 3 to 6 are diagrams for explaining a method for detecting target molecules according to the present embodiment. The method for detecting target molecules according to the present embodiment includes the following steps:
(1) adjusting the concentration of a liquid sample containing a target substance to obtain a sample solution;
(2) filling the sample solution into each of the first wells independently;
(3) releasing the target molecules from the target substance;
(4) distributing the target molecules into the second wells independently so that a single target molecule is provided per second well; and
(5) reacting the target molecules with a detection reagent to detect the target molecules.

(1) adjusting the concentration of a liquid sample containing a target substance to obtain a sample solution;
First, the concentration of a liquid sample containing the target substance is adjusted to obtain a sample solution L. This step may be abbreviated as "step (1)" hereinafter.

The sample includes, for example, a biological sample or an environmental sample. The biological sample may be serum, plasma, urine, a cell culture medium, or the like, but is not limited thereto. Examples of environmental samples may include river water, factory wastewater, and the like.

The biological sample and environmental sample contain a target substance M1 that releases target molecules to be detected. Examples of the target substance M1 include DNA, RNA, a protein, a virus, a cell, and a specific compound. Examples of RNAs include miRNA and mRNA. Examples of cells include bacteria, yeast, animal cells, plant cells, and insect cells.

The sample may contain a reaction reagent for detecting the target substance M1. That is, a biological sample or an environmental sample and a reaction reagent for detecting the target molecules contained therein may be mixed in a tube or the like, and the resulting mixture as a sample solution L may be contained in the first wells W1 of the fluidic device 100. Alternatively, first, a sample solution L not containing the reaction reagent may be injected into the fluidic device to contain the target substance in the first wells W1. Then the reaction reagent for detecting the target molecules may be contained in the first wells W1 of the fluidic device 100. The reaction reagent may be, for example, a buffer substance, an enzyme, a substrate, an antibody, or an antibody fragment. For example, when the target substance M1 is a nucleic acid, an enzyme is selected according to the biochemical reaction such as enzymatic reaction to be caused between it and the template nucleic acid associated with the target substance M1. The biochemical reaction to the template nucleic acid is, for example, a reaction such that signal amplification occurs under conditions in which the template nucleic acid is present. When the target substance M1 is a protein, an enzyme may be bound to an antibody to cause an enzymatic reaction for detecting the target substance M1. In this case, the reaction reagent (detection enzyme) may be HRP, alkaline phosphatase, β-galactosidase, or the like.

The reaction reagent is selected depending on the detection reaction to be caused. Specific examples of detection reactions include invasive cleavage assay (ICA), loop-mediated isothermal amplification (LAMP) (registered trademark), the 5' to 3' nuclease method (TaqMan (registered trademark) method), and the fluorescent probe method.

The sample may contain a surfactant. When the sample contains a surfactant, it may facilitate droplet formation in the second wells. Further, when the sample contains a surfactant, it becomes easier to replace the liquid in the fluidic device when sealing the first and second wells as described later.

Examples of surfactants include Triton-X100 (also referred to as polyethylene glycol mono-4-octylphenyl ether (n = about 10)), sodium dodecyl sulfate, Nonidet P-40 (also referred to as octylphenoxypoly(ethyleneoxy)ethanol), and Tween 20 (also referred to as polyoxyethylene sorbitan monolaurate).

The concentration of the surfactant in the total volume of the sample is preferably 0.0011 g/L or more and 55.5 g/L or less (0.0001 to 5% by volume [v/v]), more preferably 0.0111 g/L or more and 22.2 g/L or less (0.001 to 2% by volume), and even more preferably 0.111 g/L or more and 11.1 g/L or less (0.01 to 1% by volume).

When the concentration of the surfactant in the total volume of the sample is 55.5 g/L or less, there is little adverse effect on the reaction in the subsequent detection of the target substance M1.

The concentration of the sample is adjusted based on the volume of the fluidic device 100 used so that one unit of the target substance M1 is placed per first well W1. The concentration of the sample may be determined by conducting a preliminary experiment using the fluidic device 100.

(2) filling the sample solution into each of the first wells independently;
Next, the sample solution L whose concentration has been adjusted is filled into each of the first wells W1 independently This step may be abbreviated as "step (2)" hereinafter.

First, as shown in Fig. 3, the sample solution L containing the detection reagent is injected into the internal space S through the inlet 121. As the sample solution L flows through the internal space S (also referred to as the flow channel FC), the sample solution L is filled into the first wells W1 exposed to the internal space S.

After the internal space S and all of the first wells W1 are filled with the sample solution L, the sample solution L that has passed through the flow channel FC is discharged from the outlet 122.

By adjusting the concentration of the sample solution L in advance, one unit of the target substance M1 is filled in one first well W1. Specifically, when the volume of the internal space S (the volume of the sample solution L injected into the internal space S) and the total number of first wells W1 are known, the concentration of the sample solution L is adjusted so that the number of units of the target substance M1 injected into the internal space S is, for example, 1/10 or less of the total number of first wells W1. By doing so, one or fewer units of the target substance M1, that is, zero or one unit of target substance M1 is filled into one first well W1.

A means for introducing the target substance M1 into the first wells W1 is not specifically limited, and an appropriate means for the target substance M1 to be detected can be used. For example, the target substance M1 may precipitate by its own weight in the fluidic device 100 (specifically, in the flow channel FC) and distributed to the first wells W1.

A reagent that captures the target substance M1 (also referred to as a capture substance) may be used, and a target substance M1 that does not easily precipitate by its own weight may be delivered after binding it to the capture substance. As another example, the capture substance may be immobilized in the first wells W1 in advance so that the capture substance captures the target substance M1 delivered together with the sample solution L thereafter. This improves the efficiency of introducing the target substance M1 into the second wells W2.

The reaction for bonding the capture substance to the target substance M1 can be caused at any point. For example, this may be caused by bringing the target substance M1 into contact with the capture substance in a sample tube before preparing the sample solution.

Alternatively, the capture substance may be introduced into the first wells W1, and then the target substance M1 may be introduced into the first wells W1 so that the capture substance and the target substance M1 are brought into contact with each other in the first wells W1.

The capture substance is a substance capable of capturing the target substance M1. The capture substance may be, for example, a binder of a solid phase and a specific binding substance for the target substance M1. The capture substance may be a nucleic acid or a cell.

Examples of the specific binding substance include antibodies, antibody fragments, and aptamers. Examples of the antibody fragments include Fab, F(ab')2, Fab', single chain antibodies (scFvs), disulfide stabilized antibodies (dsFvs), dimeric V region fragments (diabodies), and peptides including CDRs. The antibodies may be monoclonal or polyclonal. Alternatively, the antibodies may be commercially available antibodies.

When the target substance M1 contains a sugar chain, the specific binding substance may be a lectin. When the target substance M1 includes a lipid membrane, the specific binding substance may be a substance that can bind to a lipid membrane. Examples of substances that can bind to lipid membranes include hydrocarbons such as hexanediol and membrane proteins such as transmembrane proteins. An example of a membrane protein is α-hemolysin.

The solid phase of the capture substance may be particles, membranes, substrates, or the like. One, two, or more types of specific binding substances may be used for the target substance M1. For example, there may be three, four, five, or more types of specific binding substances.

The particles are not specifically limited, but may be polymer particles, magnetic particles, glass particles, or the like. The particles are preferred to undergo surface treatment to avoid nonspecific adsorption. In order to immobilize the specific binding substance, it is preferred to use particles having a functional group such as a carboxyl group on the surfaces. More specifically, Magnosphere LC300 (product name) manufactured by JSR Corporation, or the like can be used.

The method of immobilizing the specific binding substance on the solid phase is not specifically limited, but may be a method using physical adsorption, a method using chemical bonding, a method using avidin-biotin binding, a method using binding of protein G or protein A to an antibody, or the like.

The method using physical adsorption may be a method of immobilizing the specific binding substance on particle surfaces using hydrophobic interaction or electrostatic interaction.

The method using chemical bonding may be a method using a cross-linking agent. For example, if the particle surfaces have a hydroxyl group, the carboxyl group of the specific binding substance may be reacted with a cross-linking agent to obtain an active ester, and then the hydroxyl group and the ester group may be reacted with each other, so that the specific binding substance can be immobilized on the particle surfaces.

It is preferred to provide spacers between the specific binding substance and the particle surfaces so as not to inhibit the ability of the specific binding substance to recognize the target substance M1.

When the target is a nucleic acid, the capture substance may be a nucleic acid that forms a complementary strand to the target.

As another example, when a virus is used as the target substance M1, a cell to which the virus can be attached (that is, a cell having a virus receptor) can be used as the capture substance.

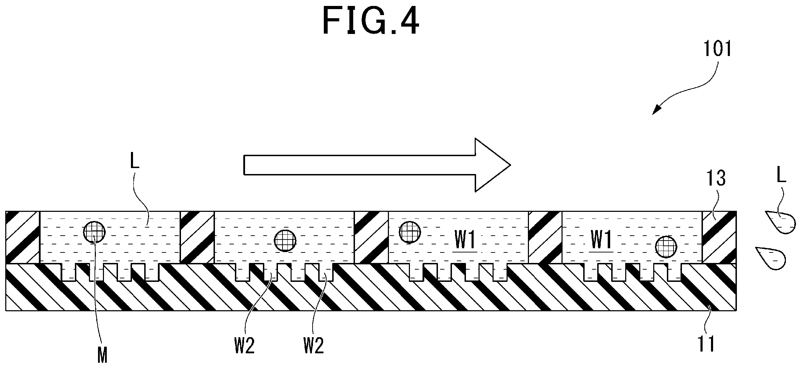

Next, as shown in Fig. 4, the cover member 12 is removed, and the part of the sample solution L above the top 13a of the wall member 13 is wiped away so that the sample solution L independently filled in each first well W1. As a result, the target substance M1 is distributed so that there is a single independent unit of the target substance M1 per first well W1.

This makes it possible to quantify the target substance M1 filled in the first wells W1. Specifically, if the target substance M1 is a nucleic acid, a detection reagent for detecting the target substance M1 can be added to measure the concentration of the target substance M1. If the target substance M1 is a cell, it can be stained using a cell staining reagent so that the number of cells can be counted.

In Fig. 4, the cover member 12 is removed, and the part of the sample solution L above the top 13a of the wall member 13 is wiped away so that the one unit of the target substance M1 in each first well W1 becomes isolated. However, the present invention is not limited to this.

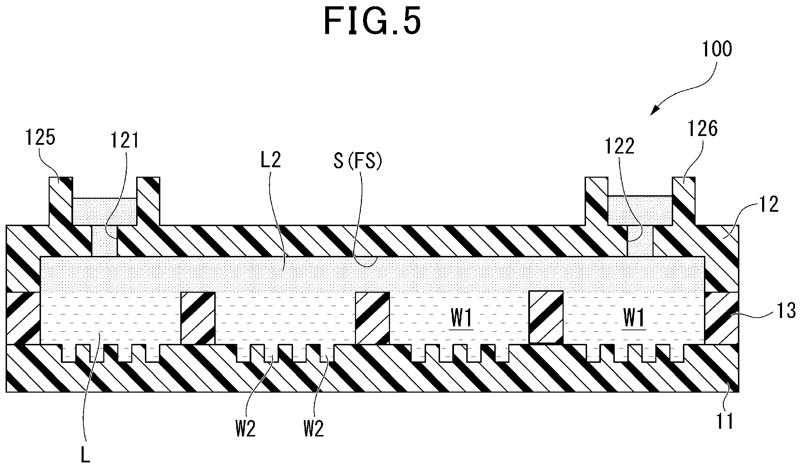

For example, as shown in Fig. 5, a sealing liquid L2 may be injected from the inlet 121 to the flow channel FC after filling the internal space S with the sample solution L. The sealing liquid L2 injected into the flow channel FC flows over the first wells W1 in the internal space S, flushing and replacing the part of the sample solution L filled in the internal space S that is not contained in the first wells W1.

The sealing liquid L2 is preferably an oily solution, and more preferably an oil. Examples of the oil that can be used include fluorinated oils, silicone oils, hydrocarbon oils, and mixtures thereof.

More specifically, FC-40 (product name) (CAS Number: 86508-42-1) manufactured by SIGMA, or the like can be used. FC-40 is a fluorinated aliphatic compound (fluorinated oil) with a specific gravity at 25°C being 1.85 g/mL.

Thus, the sealing liquid L2 individually seals the first wells W1 so that the first wells W1 containing the sample solution L become independent reaction spaces. The part of the sample solution L replaced by the sealing liquid L2 and excess sealing liquid L2 are discharged from the outlet 122.

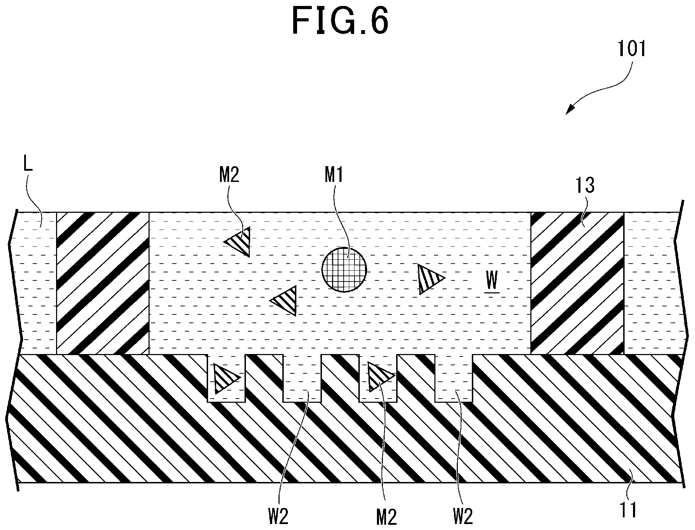

(3) releasing the target molecules from the target substance;
Next, as shown in Fig. 6, target molecules M2 are released from the target substance M1 distributed to each first well W1. This step may be abbreviated as "step (3)" hereinafter.

The method for releasing the target molecules M2 from the target substance M1 is selected depending on the type of the target substance M1. For example, when the target substance M1 is DNA or RNA, a plurality of fragments (that is, target molecules) may be obtained from one fragment by ultrasonication or enzymatic degradation.

When the target substance M1 is an aggregate formed by hybridization between nucleic acids, the fluidic device may be heated to separate the individual nucleic acids through thermal denaturation.

When the target substance M1 is a protein aggregate, the aggregate may be loosened by ultrasonic irradiation, an enzyme that degrades proteins may be used, or a reagent that breaks down protein aggregates may be added.

If the target substance M1 is a cell, the cell may be disrupted by ultrasonic waves, or the cell membrane may be disrupted by electrical stimulation.

A specific example of a method for achieving this is as follows.

First, beads are placed in the first wells W1. The beads may be placed in the first wells W1 in advance, or may be added to a solution containing cells and then placed in the first wells W1 together with the cells. The diameter of the beads is not particularly limited as long as they do not impair the effects of the present invention and can be placed in the first wells W1. The material of the beads may be silica or a magnetic material.

The diameter of the beads is preferably 50 nm to 500 µm, more preferably 100 nm to 100 µm, and even more preferably 500 nm to 10 µm.

It is preferable that at least 10 beads are placed in a first well W1.

Next, in a state where both the cells as the target substance and the beads are contained in the first wells W1, the first wells W1 are isolated in step (2), and the cells are cultured. After culturing the cells for a certain period of time, ultrasound is applied to the fluidic device 100. This causes the beads to vibrate within the first wells W1 and disrupt the cell walls of the cells and release the contents of the cells into the first wells W1.

If the target substance M1 is a cell, the cell membrane may be dissolved using a reagent that dissolves cell membranes.

A specific example of a method for achieving this is as follows.

First, a solution containing cells is mixed with a reagent that dissolves cell membranes (cell lysis solution). The solution containing the cells and the cell lysis solution may be mixed before being injected into the fluidic device 100, or may be mixed within the fluidic device 100.

If the solution containing cells is to be mixed with the cell lysis solution in the fluidic device 100, the solution containing cells may be injected into the first wells W1, and then the cell lysis solution may be injected into the first wells W1. The cell lysis solution may be added to the first wells W1 by adding it to the fluidic device 100 at a flow rate that does not cause the cells placed in the first wells W1 to leave the first wells W1. Alternatively, the cells may be immobilized in the first wells W1.

The cells may be immobilized in the first wells W1 by binding a cell capture substance to the first wells W1, or by placing particles to which a capture substance is bound in the first wells W1. The "capture substance" may be an antibody that binds to the cell. If particles to which a capture substance is bound is used, it is preferable to use magnetic beads as the particles to retain them in the first wells W1 by magnetic force when the cell lysis solution is added to the first wells W1.

The cell lysis solution may be a surfactant or a commercially available reagent.

After the cells and the cell lysis solution are added to the first wells W1, the first wells W1 are isolated in step (2) and the cell membrane is dissolved by allowing them to react for a certain period of time. This allows the contents of the cells to be released into the first wells W1.

It is also possible to culture the cells, which are the target substance M1, for a certain period of time in the first wells W1, and detect secretions such as cytokines from the cells as the target molecules M2.

Some of the released target molecules M2 are distributed to some of the second wells W2 provided at the bottom of the first wells W1. Typically, the amount of the released target molecules M2 is very small relative to the volume of the first well W1. Therefore, stochastically, one or fewer target molecules M2, that is, zero or one target molecules M2 are provided in each second well W2.

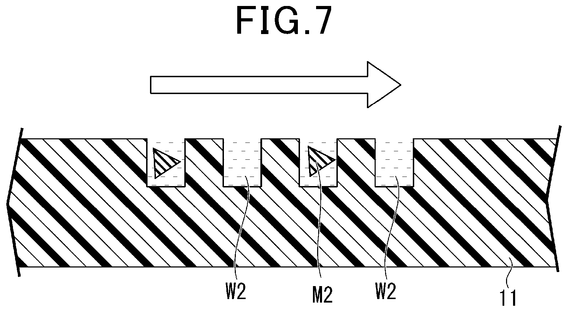

(4) distributing the target molecules into the second wells independently so that a single target molecule is provided per second well; and
Next, as shown in Fig. 7, the wall member 13 is removed, and the part of the sample solution L above the upper surface 11a of the well plate 11 is wiped away so that the sample solution L is filled in each second well W2 independently. As a result, the target molecules M2 are distributed so that there is a single independent target molecule M2 per second well W2. This step may be abbreviated as "step (4)" hereinafter.

There are various methods for filling the sample solution L into the second wells W2.

The wall member 13 may be removed by cutting the wall member 13, or by forming the wall member 13 with a resin material and dissolving the wall member 13 to remove it. In these cases, it is preferable that the wall member 13 is composed of two parts, namely, a ring-shaped first wall member provided on the outer edge of the well plate 11, and a second wall member provided on the inner part of the well plate 11. If the wall member 13 is cut, it is preferable that only the second wall member is cut. If the wall member 13 is dissolved, it is preferable to form the first and second wall members using different materials, and dissolve only the second wall member.

Alternatively, the sample solution L may be filled into the second wells W2 without removing the wall member 13.

For example, the part of the sample solution L above the upper surface 11a of the well plate 11 may be evaporated so that the sample solution L is filled in each second well W2 independently. Alternatively, the sample solution L1 may be removed by suction until the liquid level of the sample solution L1 is in the first wells W1. The sealing liquid L2 may be delivered through the inlet 121 to the flow channel FC after that so that the sample solution L is filled into the second wells W2.

It is also possible to deliver the sealing liquid L2 through the inlet 121 to the flow channel FC with the first wells W1 filled with sample solution L, thereby replacing the sample solution L in the first wells W1 with the sealing liquid L2 and sealing the second wells W2 with the sealing liquid L2. Computer simulation suggests that when the sealing liquid L2 is injected at a certain flow rate or faster, the sealing liquid L2 flows over the first wells W1 in the internal space S as in Fig. 5, whereas when the flow rate is lower, the sealing liquid L2 delivered from the inlet 121 can enter the first wells W1 and replace the sample solution L in the first wells W1. It is preferable that an appropriate flow rate is determined beforehand through preliminary experiments because the flow rate is affected by the diameter of the first wells W1, the type of the sealing liquid L2, and the materials of the well plate 11 and wall member 13.

In addition, if the sealing liquid L2 is used to fill the sample solution L1 independently into the first wells W1 in the above-described step (2), a sealing liquid having a lower viscosity than the sealing liquid used in step (2) may be used in this step. If the sealing liquid used in step (2) flows over the first wells W1 in the internal space S, a sealing liquid having a lower viscosity than the sealing liquid used in step (2) may be delivered to the flow channel FC in this step, so that the sealing liquid L2 enters the first wells W1 and replaces the sample solution L in the first wells W1.

If the sample solution L in the first wells W1 is to be replaced by the sealing liquid L2 as described above, it is possible to facilitate the injected sealing liquid reaching the bottom surfaces of the first wells W1 by widening the opening so that the wall is inclined relative to the bottom surface of each first well W1.

To facilitate this operation, the well plate 11 and the wall member 13 are preferably made of different materials.

For example, forming the well plate 11 from a hydrophilic material makes it difficult for the sealing liquid to enter the second wells W2 and facilitates sealing with the sealing liquid L2.

If the wall member 13 is removed by cutting, the well plate 11 may be formed using a material harder than that of the wall member 13 so as to precisely cut away only the wall member 13. An example of such a combination of materials is an inorganic material for the well plate 11 and a resin material for the wall member 13.

### (5) Detecting target molecules

Next, the target molecules are reacted with a detection reagent to detect the target molecules. This step may be abbreviated as "step (5)" hereinafter. The detection reagent may be contained in the sample solution L in advance, or a liquid containing the detection reagent may be introduced into the fluidic device 100 from outside at any stage.

An example of the detection method is to cause a signal amplification reaction inside the sealed second wells W2. In the second wells W2, a signal originating from the detection reagent is amplified for detection.

Examples of signals include fluorescence, coloring, electric potential change, pH change, and the like.

The signal amplification reaction may be, for example, a biochemical reaction, and more specifically, an enzymatic reaction. As an example, the signal amplification reaction is an isothermal reaction in which, while a reagent solution containing an enzyme for signal amplification is accommodated in the second wells W2, the fluidic device 100 is maintained at a constant temperature condition under which a desired enzyme activity is obtained, for example, 60°C or more, and preferably approximately 66°C, for a predetermined period of time, for example, at least 10 minutes, and preferably approximately 15 minutes.

When the target molecule M2 is a nucleic acid, specific examples of the signal amplification reaction include an ICA reaction such as an Invader (registered trademark) method, loop-mediated isothermal amplification (LAMP) (registered trademark), the 5' to 3' nuclease method (TaqMan (registered trademark) method), and the fluorescent probe method. It is considered that these methods can cause a signal amplification reaction even if the solution contains the above-described surfactant (which may be contained in a sample or cell lysis solution).

If the cell lysis solution inhibits the signal amplification reaction, the solution in the second wells W2 may be replaced when the reaction is triggered. In that case, the target molecules M2 may be immobilized in the second wells W2 before adding the detection reagent from the outside.

To immobilize the target molecules in the second wells W2, a substance that captures the target molecules M2 may be bound to the second wells W2, or magnetic particles bound to a compound that captures the target molecules M2 may be added to the second wells W2, and the magnetic particles may be immobilized by magnetic force.

A particularly preferable signal amplification reaction is ICA reaction. In an ICA reaction, signal amplification progresses through two reaction cycles which are (1) complementary binding between nucleic acids and (2) recognition and cleavage of a triple-stranded structure by enzymes. In such a signal amplification reaction, influence of contaminants other than the target molecule M2 on the reaction cycle is small. Therefore, even if there are various components (for example, contaminants) in the second wells W2 other than the target molecule M2, the target molecule M2 can be accurately detected by using the ICA reaction.

For example, when an ICA reaction is used for the signal amplification reaction, the sample solution L includes a reaction reagent and a template nucleic acid required for the ICA reaction. When the biochemical reaction in the reaction step is an ICA reaction, in second wells W2 containing the target molecule M2, a fluorescent substance is released from the quenching substance, which results in a specified fluorescence signal being emitted corresponding to the excitation light.

The target molecule M2 can also be detected by binding a substance specifically binding to the target molecule M2 (also called specific binding substance) to the target molecule M2, and detecting the specific binding substance that has been bound. For example, when the target molecule M2 is a protein, ELISA can be used for detection. More specifically, the detection may also be performed by, for example, sandwich ELISA using the principle of FRET.

In performing sandwich ELISA using the principle of FRET, first, a first specific binding substance (e.g., antibody) labeled with a first fluorescent substance (also called donor), and a second specific binding substance labeled with a second fluorescent substance (also called acceptor) and having a light-absorbing wavelength that overlaps a fluorescence wavelength of the first fluorescent substance are prepared.

Then, the target molecule M2 (e.g., an antigen) is brought into contact with both the first specific binding substance and the second specific binding substance to form a composite. Once a composite is formed, a distance between the donor and the acceptor decreases, and the fluorescence wavelength of the acceptor can be detected by irradiation at the excitation wavelength of the donor. Alternatively, the specific binding substance may be labeled with a nucleic acid fragment, and then the nucleic acid fragment can be detected by the ICA reaction.

Examples of the specific binding substance include those similar to the specific binding molecules for the structures, which will be described later, such as antibodies, antibody fragments, and aptamers. In order to detect a specific binding substance bound to the target molecule M2, the specific binding substance may be directly or indirectly labeled by, for example, an enzyme such as horseradish peroxidase (HRP). When two or more specific binding substances are used, each of the specific binding molecules are labeled so that each can be identified.

A signal observation method may be selected from known appropriate methods depending on the type of the signal to be observed. For example, when bright field observation is performed, white light is irradiated in a vertical direction to the substrate provided with the well array. When fluorescence signal observation is performed, excitation light corresponding to the fluorescent substance is irradiated into the well via the bottom of the well to observe fluorescence emitted from the fluorescent substance. An image of the entirety or a part of the observed well array is acquired and stored, and then image processing is performed by a computer system.

According to the fluidic device 100 configured as above, target molecules released from the target substance in the solution can be detected with high sensitivity.

In addition, according to the above-described method for detecting target molecules, the target molecules can be detected with high accuracy.

Note that, although step (1) is performed in this embodiment, step (1) may be omitted. When step (1) is not performed, after filling the first wells with the sample solution, first wells W1 containing one unit of the target substance M1 may be extracted from the plurality of first wells in step (3), followed by steps (4) and (5).

Visualizing the target substance M1 facilitates extracting the first wells containing one unit of the target substance M1. When the target substance M1 is a cell, a possible visualization method is to bring the cell into contact with a staining reagent in advance.

When the target substance M1 is a nucleic acid, a possible visualization method is to mix the target substance M1 with a known DNA staining reagent before injecting it into the fluidic device 100. After staining, only the nucleic acid may be recovered by a known method, and the recovered nucleic acid may be added to the sample solution L1 again, which may then be injected into the fluidic device 100 and placed in the first wells W1.

When the target substance M1 is a nucleic acid (for example, genomic DNA or chromosome), an ICA reaction may be triggered in the first wells W1 to extract first wells W1 that contain one unit of the target substance M1.

Specifically, a fluorescent substance different from the fluorescent substance used in step (5) is contained in a sample solution L1 containing a nucleic acid, and the sample solution L1 is then injected into the fluidic device. Next, after placing the target substance M1 in each first well W1 in step (2), an ICA reaction is triggered in the first wells W1. The fluorescence intensity after a certain reaction time is measured to identify the first wells W1 that contain one nucleic acid molecule.

If the number of glowing wells is a certain number or less (specifically, 1/10 of the total number of first wells), it can be considered that most of the glowing wells contain only one nucleic acid molecule based on the Poisson distribution.

After confirming the first wells W1 containing one nucleic acid molecule, the nucleic acid is fragmented by restriction enzyme treatment in the first wells W1, and a resulting DNA fragment (that is, the target molecule) is detected in step (5). By selecting the type of restriction enzyme so that a specific gene region is contained in a DNA fragment in a one-to-one correspondence, and detecting the "specific gene region" in step (5), copy number variation (CNV) analysis can be performed.

After visualization using the above method, a microscopic picture of the first wells W1 containing the target substance M1 may be taken, and the captured image may be analyzed to extract the first wells W1 containing one unit of the target substance M1.

### [Second Embodiment]

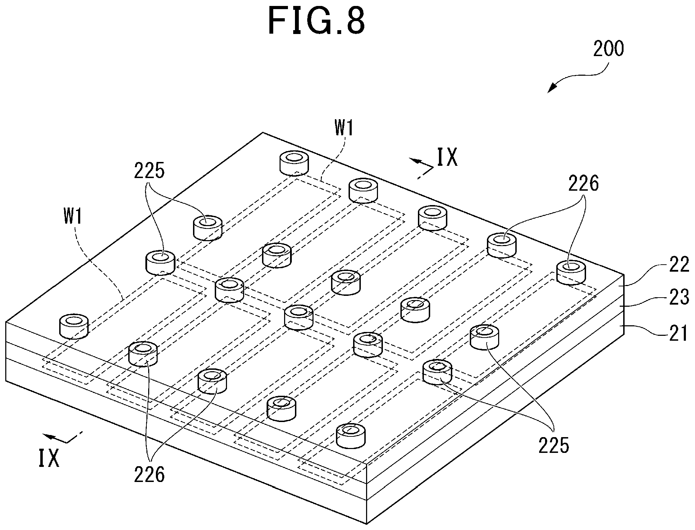

Figs. 8 to 11 are diagrams for explaining a fluidic device according to a second embodiment of the present invention. In this embodiment, constituent elements that are also used in the first embodiment are designated by the same reference numerals, and detailed description thereof is omitted.

### [Fluidic Device]

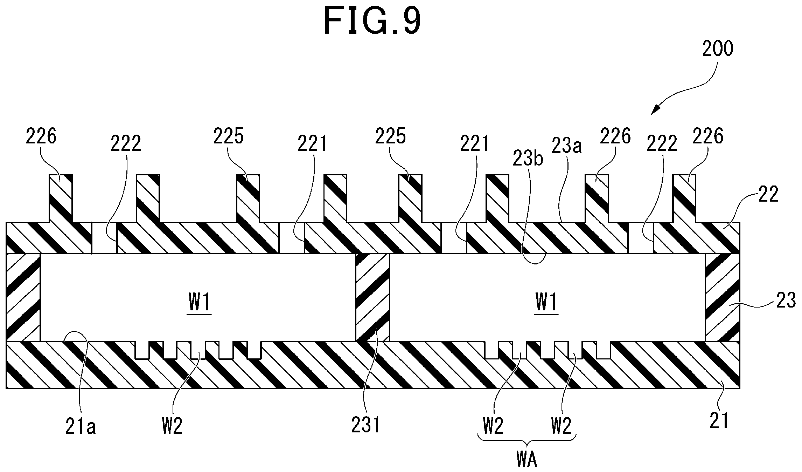

Fig. 8 is a schematic perspective view illustrating a fluidic device according to the present embodiment. Fig. 9 is a cross-sectional view taken along the line IX-IX of Fig. 8.

A fluidic device 200 includes a well plate (also called a substrate) 21, a cover member 22, and a wall member 23. The fluidic device 200 is used as a reaction vessel configured to contain a sample in an internal space S. In the reaction vessel, target molecules are released from a target substance in the sample, and a reaction for detecting the target molecules is induced.

The fluidic device 200 has a plurality of first wells W1 surrounded by the well plate 21, the cover member 12, and the wall member 13, and a plurality of second wells W2 provided in an upper surface 21a of the well plate 21 corresponding to the bottoms of the first wells W1.

The following describes these components sequentially.

### (Well Plate)

The well plate 21 is a plate-like member having a rectangular shape in plan view. The term "plan view" refers to a view from the vertical direction of the upper surface 21a of the well plate. The upper surface 21a of the well plate 21 is provided with a plurality of wells (also called second wells) W2.

The example materials for the well plate 11 of the first embodiment can be used to form the well plate 21.

### (Wall Member)

The wall member 23 has a closed ring shape in plan view, and is placed on the upper surface 21a of the well plate 21.

The wall member 23 is sandwiched between the well plate 21 and the cover member 22 to be integrated with them and form the fluidic device 200. The space surrounded by the well plate 21, the cover member 22, and the wall member 23 is the internal space S in which a liquid sample is contained.

The wall member 23 has a side wall 231 provided on the outer edge of the well plate 21 and an internal partition wall 232 provided on the inner part of the well plate 21. The wall member 23 serves as a wall surface of the internal space S, and also serves as a spacer between the well plate 21 and the cover member 22.

The space surrounded by the well plate 21, the wall member 23 (the side wall 231 and the internal partition wall 232), and the cover member 22 is the internal space S, and is the first wells W1. The fluidic device 200 has a plurality of first wells W1 (2 × 5 = 10 in Fig. 8). The first wells W1 have a rectangular shape when viewed in plan view. The first wells W1 all extend in the same direction.

The material of the wall member 23 may the same as that of the well plate 21 described above.

### (Cover Member)

The cover member 22 has the same contour as the well plate 21 when viewed in plan view. The cover member 22 faces the well plate 21 and is in contact with the wall member 23.

The cover member 22 includes a plurality of through holes passing therethrough in the thickness direction. The through holes are located at opposite ends of each first well W1. One of the through holes of a first well W1 is an inlet 221 used to inject a liquid into the internal space S of the fluidic device 200, and the other through hole is an outlet 222 used to discharge the liquid from the internal space S.

The inlet 221, the internal space S, and the outlet 222 are connected in this order to form a flow channel FC as a whole. The fluidic device 200 allows a liquid to flow through the flow channel FC to cause a target substance detection reaction.

The upper surface 22a of the cover member 22 is provided with a cylindrical injection port 225 surrounding the inlet 221. The injection port 225 is connected with the inlet 221. For example, the injection port 225 is used to connect a syringe filled with a liquid to fill the internal space with the liquid using the syringe.

Similarly, the upper surface 22a of the cover member 22 is provided with a cylindrical discharge port 226 surrounding the outlet 222. The discharge port 226 is connected with the outlet 222. The discharge port 226 is used, for example, to connect a tube through which the liquid flows when the liquid is removed from the internal space S.

The injection ports 225 of adjacent first wells W1 are located adjacent to each other and arranged in two rows and five columns along the aligned first wells W1.

The example materials for the well plate 21 described above can be used to form the cover member 22. The material of the cover member 22 may or may not be the same as the material of the well plate 21.

### <Method for Detecting Target Molecule>

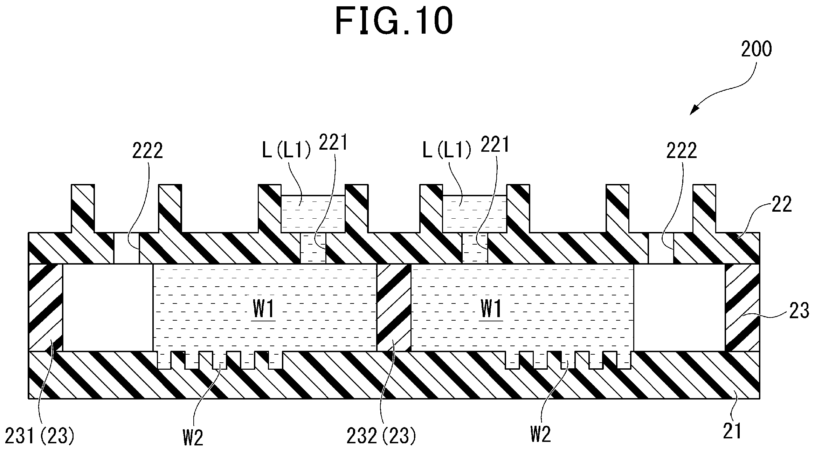

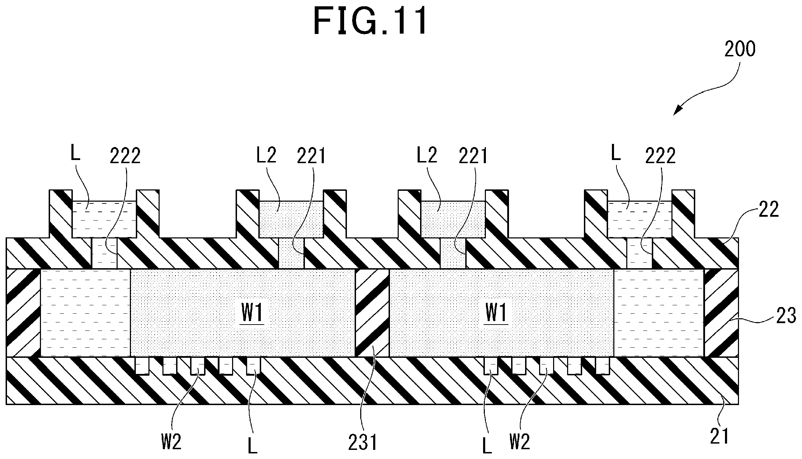

Figs. 10 and 11 are diagrams for explaining a method for detecting target molecules according to the present embodiment.

First, as shown in Fig. 10, a sample solution L whose concentration has been adjusted in the same manner as in the first embodiment is injected into the internal space S (the first wells W1) from the inlet 221. By adjusting the concentration in advance, one unit of the target substance (not shown) is distributed to each first well W1.

After the internal space S is filled with the sample solution L, the sample solution L that has passed through the flow channel FC is discharged from the outlet 222.

Next, similarly to the first embodiment, target molecules (not shown) are released from the target substance in the first wells W1. The released target molecules are filled into the second wells W2 so that there is one or fewer target molecules, that is, zero or one molecule in each second well W2.

Then, as shown in Fig. 11, the sealing liquid L2 is delivered to the flow channel FC from the inlet 221. The sealing liquid L2 injected into the flow channel FC flows through the internal space S along the upper surface 11a, washing away and replacing the part of the sample solution L injected into the flow channel FC that is not contained in the second wells W2.

Thus, the sealing liquid L2 individually seals the second wells W2 so that the second wells W2 containing the sample solution L become independent reaction spaces. When the flow channel FC is filled with the sealing liquid L2, excess sealing liquid L2 is discharged from the outlet 222.

After that, a target molecule detection reaction is caused in the second wells W2 as in the first embodiment.

According to the fluidic device 200 configured as above, target molecules released from the target substance in the solution can be detected with high sensitivity.

In addition, according to the above-described method for detecting target molecules, the target molecules can be detected with high accuracy.

### [Third Embodiment]

A method for detecting a target molecule according to another aspect that uses the fluidic device disclosed in the first embodiment will now be described.

A method for detecting a target molecule of the present embodiment uses a fluidic device having wells, the method including: placing a reaction solution in the wells for reaction between the target molecule, alkaline phosphatase immobilized on a binder that binds to the target molecule, and a substrate that changes visually when dephosphorylated by the alkaline phosphatase; and visualizing and detecting a presence of the target molecule contained in the wells by using a dephosphorylation reaction of the substrate by the alkaline phosphatase in the wells, in which, at a start of the step of visualizing and detecting the presence of the target molecule, a concentration of the substrate in the reaction solution contained in the wells is 80 µmol/L or more and 800 µmol/L or less.

As another aspect, the present embodiment provides a method for detecting a target molecule using a fluidic device having a well, including: providing a target molecule to the well; providing alkaline phosphatase to the well; providing a substrate for the alkaline phosphatase to the well; and detecting the target molecule in the well using the alkaline phosphatase and the substrate.

In this method, by setting the concentration of the alkaline phosphatase substrate added to the well within an appropriate range, it is possible to suppress false positive reactions caused by autolysis due to the excitation light of the fluorescence microscope. Note that the steps of providing the target molecule to the well, providing the alkaline phosphatase to the well, and providing the substrate for the alkaline phosphatase to the well may be performed simultaneously or separately. The target molecule, alkaline phosphatase, and substrate may be mixed and then added to the well as a single solution. The term "single solution" corresponds to "reaction solution" in this embodiment.

### (Fluidic Device)

The fluidic device disclosed in the first embodiment can be used as the fluidic device used in the embodiment of this embodiment. As an example, a case where the fluidic device 100 shown in Figs. 1 and 2 is used will be used to describe the detection of the target molecule.

The detection method in this embodiment includes providing a liquid containing alkaline phosphatase and its substrate fluorescein di-phosphate (FDP) to the second wells W2, and observing the fluidic device 100 with a fluorescence microscope.

First, the sample solution L, which is introduced into the internal space S (first wells W1) from the inlet 121 of the fluidic device 100 after having its concentration being adjusted as in the first embodiment, is as described in the first embodiment, and therefore a description thereof will be omitted.

After the internal space S is filled with the sample solution L, the sample solution L that has passed through the flow channel FC is discharged from the outlet 122.

Next, similarly to the first embodiment, target molecules (not shown) are released from the target substance in the first wells W1. The number of target molecules introduced into each second well W2 is not specifically limited, but one or fewer, that is, zero or one target molecule is preferred to be introduced into each second well W2. This makes it possible to detect single target molecules, enabling digital measurement. Not all of the second wells W2 need to be provided with a target molecule.

Examples of reaction reagents in this embodiment include a buffer substance, alkaline phosphatase as an example of an enzyme, a substrate, an antibody, and an antibody fragment. Alkaline phosphatase is referred to as "ALP" hereinafter. The antibody may be monoclonal or polyclonal. The antibody may be a full-length antibody or an antibody that has had a part of it cleaved off by enzyme treatment or the like. ALP, DNA, or the like may be bound to the antibody.

ALP is an enzyme that hydrolyzes a phosphate compound (for example, a phosphate monoester), which is a substrate, to liberate phosphate.

Examples of substrates that can be used with ALP include FDP, p-nitrophenyl phosphate (pNPP), and monofluorophosphate (MFP).

FDP and MFP are non-fluorescent substances, but become fluorescent when dephosphorylated.

pNPP is a pale white substance, but turns yellow when dephosphorylated.

By using a substrate such as FDP, MFP, or pNPP that undergoes a visual change when dephosphorylated by ALP, the presence of the target molecule can be visualized (i.e., labeled). In this case, visual changes or visualization are not limited to changes that can be seen with the naked eye, but may also be changes that can be seen, for example, using a microscope or when imaged using an image sensor.

In this embodiment, a case where FDP is used as the substrate for alkaline phosphatase will be described.

### <<Retention of Target Molecules in Second Wells> >

If the sample solution L does not contain a reaction reagent, and a reaction reagent such as an enzyme that detects the target molecule is added from the outside after the sample solution L has been injected into the fluidic device 100, the target molecule needs to be retained in each second well W2.

The target molecules may precipitate by its own weight in the fluidic device (specifically, in the flow channel) and distributed to the second wells W2. A capture substance may be used as described in the first embodiment.

After the target substance is captured in the second wells W2 using the capture substance, the second wells W2 may be washed before ALP is supplied.

If the target substance is captured by a capture substance on particles outside the fluidic device 100, the ALP immobilized to a binder described in detail below and the FDP may be supplied to a container containing the particles and the target substance outside the fluidic device 100, and then supplied to the fluidic device 100.

Regardless of whether ALP is mixed with the target substance outside the fluidic device 100 or in the first wells W1, it is necessary to wash away ALP that are not bound to the target molecules before the supply of FDP. This prevents fluorescence emission in second wells W2 with no target molecules.

FDP is supplied after the ALP that are not bound to the target molecules have been removed.

Note that there may be one or more means for capturing the target molecule. For example, there may be three, four, five, or more types of specific binding substances.

The sample solution L, and the solution (i.e., reaction solution) contained in the second wells W2 during the reaction for detecting the target molecule (i.e., during the detection process), that is, the solution containing the target molecules, ALP, and FDP, may contain a surfactant or a buffer solution. The examples listed in the first embodiment may be used as the surfactant.

The concentration of the surfactant in the total volume of the reaction solution in the wells at the start of the target molecule detection reaction is preferably 0.001% by volume or more and 5% by volume or less, more preferably 0.01% by volume or more and 1% by volume or less, and even more preferably 0.03% by volume or more and 0.1% by volume or less. When the concentration of the surfactant is 1% by volume or less, there is little effect on the reaction in the subsequent detection of the target molecules.

### <<Binding of ALP to Target Molecules>>

To detect a target molecule, ALP is immobilized to a binder that binds to the target molecules, and a fluorescence reaction is triggered in which the ALP, bound to the target molecules via the binder, dephosphorylates (that is, degrades) FDP. Therefore, the confirmation of the fluorescence reaction means that FDP and ALP have reacted, and can be considered as indirect evidence of the presence of target molecules bound to ALP.

The binder that binds to the target molecule may be a nucleic acid, an antibody, or the like.

For example, when the target molecule is a nucleic acid, the ALP may be immobilized to a binder nucleic acid (that is, a nucleic acid for detection) that is complementary to the target nucleic acid.

Alternatively, a protein may be bound in advance to a nucleic acid complementary to the nucleic acid of the target molecule, and then the complementary nucleic acid may be bound to the nucleic acid of the target molecule. By supplying ALP immobilized on an antibody (for detection) that binds to this protein, the ALP can be bound to the target molecule.

When the target molecule is a protein, the ALP may be immobilized on an antibody as a binder that binds to the target molecule.

Thus, it is possible to ensure that the ALP added to the second wells W2 binds to the target molecule by using a binder that binds to the target molecule.

In the case where ALP is immobilized on a nucleic acid or antibody as a binder, covalent bonding between an amino group and a carboxyl group may be utilized, or the ALP may be bonded via biotin and streptavidin, or the ALP may be bound using other reactions.

The binder on which ALP has been immobilized may be provided to the second wells W2 after the sample solution L, which may contain the target substance in advance, has been contained in the fluidic device 100 and the target molecules have been contained in the second wells W2. Alternatively, it may be supplied to the second wells W2 before the target substance. It is also possible to mix ALP with the sample solution L, which may contain the target substance, outside the fluidic device 100 and then supply the mixture to the fluidic device 100.

### <<Well Washing>>

After the binder on which ALP has been immobilized is supplied to the fluidic device 100, a washing solution for washing the second wells W2 may be supplied to the fluidic device 100.

By washing the second wells W2 in advance, it is possible to prevent a situation where the ALP decomposes FDP in second wells W2 that do not contain target molecules. If the second wells W2 are washed, FDP is supplied to the second wells W2 after the washing.

The washing solution may contain, for example, a blocking component such as bovine serum albumin, a surfactant, a salt, or a buffer solution.

### <<Distribution of Target Molecules to Second Wells>>

In the same manner as in the first embodiment, the target molecules are distributed independently into the second wells so that a single target molecule is provided per second well.

### <<Target Molecule Detection>>

After each second well W2 is isolated, a reaction is caused with the reaction reagent contained in the sample solution L in the second wells W2 to detect the target molecules.

As described above, the detection of target molecules utilizes the fluorescence emitted from the decomposition products of FDP. On the other hand, even when FDP is decomposed without reacting with ALP and generates decomposition products, a similar fluorescence reaction is observed. Since the confirmation of a fluorescence reaction is indirect evidence of the presence of target molecules, in such cases, it may be erroneously determined that target molecules are present, resulting in a "false positive".

Based on the idea that FDP may be photodecomposed by the excitation light emitted to confirm the presence of a fluorescence reaction, and the resulting decomposition products may lead to false positives, the inventors have conducted extensive research. As a result, they discovered that, by appropriately controlling the concentration of FDP to fall within a certain concentration range, it is possible to effectively suppress false positive reactions that are considered to be caused by the self-decomposition of FDP due to excitation light, which led them to the present invention.

In the reaction solution, which may contain ALP, FDP, and the target molecule, contained in wells 141, the concentration of FDP is in the range of 80 µM (µmol/L) to 800 µM, preferably in the range of 100 µM to 800 µM, and more preferably in the range of 200 µM to 600 µM.

These FDP concentrations refer to the concentrations of FDP at the time the detection reaction (i.e., enzymatic reaction) using ALP and FDP has started, once a liquid L110, which may contain the target molecules, ALP, and FDP have all been contained in the wells 141.

When the target molecule is a nucleic acid, first, a nucleic acid amplification reagent may be added to the reaction reagent to amplify the nucleic acid, and then the nucleic acid may be detected with ALP and FDP.

A certain reaction time is required for ALP to decompose FDP. The reaction may be performed at room temperature, or at 37°C by heating. The reaction temperature is preferably 10°C to 50°C, more preferably 20°C to 40°C, and even more preferably 25°C to 37°C. The reaction time is preferably 10 minutes to 2 hours, more preferably 20 minutes to 1 hour, and even more preferably 30 minutes to 50 minutes.

### <<Fluorescence Observation>>

After the reaction time has elapsed, FDP would be decomposed (i.e., dephosphorylated) by ALP in the second wells W2 containing the target molecules. When excitation light of a specific wavelength is emitted from a fluorescence microscope, fluorescent light is detected from the wells 141 in which FDP has been decomposed, and this property is used to detect the target molecules.

According to one mode of digital measurement using ALP, when a sample contains a plurality of target molecules, for example, when the target molecules are a protein, the detection of the target molecules can be facilitated. The present invention includes the following mode as an example.

First, a sample solution L containing a protein, which is an example of a target molecule, is added to the fluidic device 100. At this time, it is preferable to use a capture substance to capture the protein in the second wells W2.

Next, a detection solution containing ALP immobilized on an antibody, which is an example of a binder, is added to the fluidic device 100. The mixture is then left to stand for a certain period of time to allow the detection antibody and ALP to bind to the target molecules.

After the binder has been bound to the target molecules, a wash buffer is added to the fluidic device 100. The washing may be performed once, five times, or ten times. This makes it possible to remove excess binders that are not bound to the target molecules and excess ALP bound to the binders, thereby suppressing false positive reactions.

After that, a reaction reagent containing FDP, which is a substrate for ALP, is added to the fluidic device 100.

Then, each of the second wells W2 is isolated.

Once the ALP and FDP are contained and isolated in each second well W2 in this manner, they are reacted for a certain period of time at a certain temperature to cause the ALP to dephosphorylate the FDP.

After the dephosphorylation reaction by ALP, in order to confirm the number of second wells W2 containing target molecules, each second well W2 is observed under a fluorescence microscope to count the number of second wells W2 that emit fluorescent light.

The number and concentration of the target protein molecules can be calculated from the counting results.

As another example of detection of a protein, which is an example of the target molecule, a case where the steps of capturing the protein and binding the ALP are performed outside the fluidic device 100 will be described below.

First, particles with an antibody as a capture substance immobilized thereon are added to a tube, and a solution containing the protein as the target molecule is also added.

Next, a solution containing ALP immobilized on a detection antibody, which is a binder, is added to the tube. As a result, the target molecules are attached to the capture substance on the particles, and the binder and ALP are attached to the target molecules. Note that it is also possible to place and mix the particles with the capture substance immobilized thereon, the target molecules, the binder, and ALP in the tube simultaneously.

After the ALP is bound to the target molecules via the binder in this manner, the solution is removed, and an FDP reagent is added to the tube to suspend the particles.

The solution and particles in the tube are then supplied to the fluidic device 100. Instead of adding the FDP reagent to the tube, the particles may be added to the fluidic device 100 first, and the FDP solution may be injected into the second wells W2 after that.

Then, each of the second wells W2 is isolated. The mixture is left to stand at a constant temperature for a certain period of time to allow the ALP to react.

After the reaction, the number of wells emitting fluorescent light is counted using excitation light under a fluorescence microscope. The number of target molecules can thus be digitally measured.

According to this method, it is possible to suppress false positives and improve the accuracy of target molecule detection.

### (Fluidic Device Modification)

The fluidic device used in the detection method of this embodiment may be any fluidic device other than the fluidic device disclosed in the first embodiment, as long as it has a well that contains the target molecule and the like, and its mode is not particularly limited.

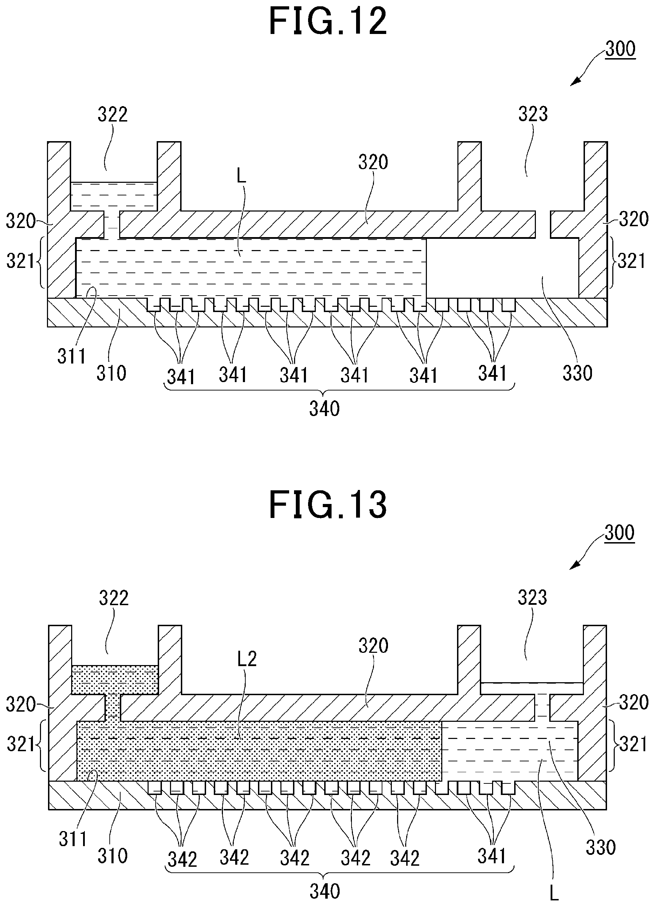

Fig. 12 is a schematic cross-sectional view illustrating a fluidic device according to a modification of the present embodiment. As shown in Fig. 12, a fluidic device 300 as one example includes a substrate 310 and a cover member 320 covering the substrate 310.

The substrate 310 may be made of a resin, for example. The type of the resin is not specifically limited, but it is preferred to be a resin resistant to a liquid L310 and a sealing liquid L2. If the signal to be detected is fluorescence, the resin is preferred to have low autofluorescence. Examples of the resin include cycloolefin polymers, cycloolefin copolymers, silicones, polypropylenes, polycarbonates, polystyrenes, polyethylenes, polyvinyl acetates, fluororesins, and amorphous fluororesins, but are not limited thereto.

The substrate 310 has a flat plate shape, and an upper surface 311 of the substrate 310 is formed with a well array 340 having a large number of wells 341 for containing a sample, a liquid, particles, and the like. Examples of the method of forming wells using a resin include injection molding, thermal imprinting, and optical imprinting.

The shape, size, and arrangement of the wells 341 are not specifically limited; however, it is preferable that one target molecule is introduced into one well 341. The wells 341 are preferably microwells of a small volume. For example, each well 341 may have a volume of approximately 10 fL to 100 pL.

In this embodiment, the large number of wells 341 have the same shape and size. The same shape and size may refer to the same shape and volume required for the digital measurement, accepting a manufacturing error variation.

The wells may have the same shape as the second wells W2 described in the first embodiment.

Each well 341 may have a diameter of, for example, approximately 1 µm to 100 µm. Each well 341 may have a depth of, for example, approximately 1 µm to 100 µm. The arrangement of the wells 341 is not specifically limited, and may be arranged, for example, in a triangular lattice shape or a square lattice shape, or may be randomly arranged.

The cover member 320 has a protrusion part 321 that protrudes downward on its edge. The protrusion part 321 has a ring shape having the same shape as the contour of the substrate 310 in plan view, and is in contact with the peripheral part of the substrate 310. Therefore, a flow channel 330 is formed between the substrate 310 and the cover member 320. The lower end of the protrusion part 321 may be welded or adhered to the substrate 310.

The flow channel 330 functions as a path for delivering the sample solution L and the sealing liquid L2 described later. That is, the sample solution L and the sealing liquid L2 are introduced into the fluidic device 300 through the flow channel 330.

The material of the cover member 320 is preferred to be a resin with low autofluorescence, examples of which include thermoplastic resins such as cycloolefin polymers and cycloolefin copolymers.

The cover member 320 may be formed of a material that does not transmit light with a wavelength detected in fluorescence observation for signals and light with wavelengths near that wavelength, or a material that is completely opaque to light. Therefore, for example, the cover member 320 may be made of a thermoplastic resin with an addition of carbon, metal particles, etc.

The shape, structure, capacity, and the like of the flow channel 330 are not particularly limited, but the flow channel 330 may have a height of, for example, 100 µm or less.

The cover member 320 is formed with an introduction port 322 for supplying various liquids, particles, and the like to the flow channel 330, and a discharge port 323 for discharging liquids and the like from the flow channel 330.

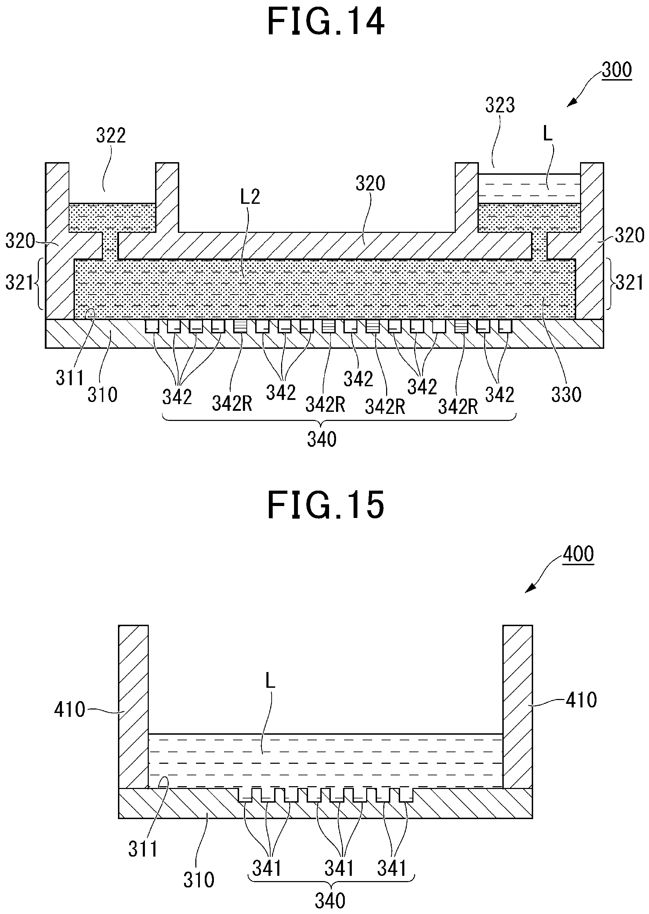

Fig. 15 is a schematic cross-sectional view of a fluidic device 400 according to another preferred embodiment of the present invention.

As shown in Fig. 15, the fluidic device 400 includes the substrate 310 and a wall member 410 extending upwards from an edge part of the upper surface 311 of the substrate 310.

Similarly to the fluidic device 300 shown in Fig. 12, the substrate 310 is formed with the well array 340 including the large number of wells 341. Target molecules can still be detected even when the fluidic device 400 in which the wells 341 have no cover over them is used.

Note that, in the detection method of this modification, after a liquid containing ALP and its substrate FDP is placed in the wells 341, the openings of the wells 341 are sealed with the sealing liquid L2, and then the fluidic device 300 or 400 is observed under a fluorescence microscope.

The example configurations of the fluidic devices 300 and 400 have been described. Next, the detection of target molecules using the fluidic device 300 shown in Figs. 12 to 14 will be described.

First, as shown in Fig. 1, a sample solution L, whose concentration has been adjusted in the same manner as in the first embodiment, is introduced from the introduction port 322 of the fluidic device 300 and delivered to the flow channel 330.

After the flow channel 330 is filled with the sample solution L, the sample solution L that has passed through the flow channel 330 is discharged from the discharge port 323.

The number of target molecules introduced into each well 341 is not specifically limited, but one or fewer, that is, zero or one target molecule, are preferably introduced into one well 341. This makes it possible to detect single target molecules, enabling digital measurement. Note that not all of the wells 341 of the well array 340 need be provided with a target molecule. The sample solution L is as described in this embodiment.

Then, the above-described <<Retention of Target Molecules in First Wells> >, < <Binding of ALP to Target Molecules>>, and <<Well Washing>> are carried out.

### <<Introduction of Sealing Liquid>>

Fig. 13 is a schematic cross-sectional view showing a state where the sealing liquid L2 is being supplied to the fluidic device 300 shown in Fig. 12. Fig. 14 is a schematic cross-sectional view showing a state where the sealing liquid L2 has been supplied to the fluidic device shown in Fig. 12.

Once the sample solution L that may contain target molecules, ALP, and FDP are contained in the wells 341, the sealing liquid L2 is supplied to the flow channel 330 through the introduction port 322.

The sealing liquid L2 flows through the flow path 330 from left to right in the drawing as shown in Fig. 13, and once the flow path 330 is filled with the sealing liquid L2, it rises toward the discharge port 323.

The sealing liquid L2 is a liquid that can form droplets (also referred to as microdroplets) by sealing the liquid introduced into the multiple wells 341 individually so that they do not mix with each other. The sealing liquid L2 used in the first embodiment can be used in this example.

The sealing liquid L2 delivered to the flow channel 330 may wash away and replace the part of the sample solution L supplied to the flow channel 330 and not accommodated in the wells 341. As a result, the sealing liquid L2 individually seals the plurality of wells 341 to provide the wells 341 as independent reaction spaces (micro compartments 342).

Once the flow channel 330 is filled with the sealing liquid L2, excess sealing liquid L2 is discharged from the discharge port 323. Fig. 14 shows a state in which all the wells 341 of the well array 340 are sealed with the sealing liquid L2, forming sealed wells (i.e., micro compartments) 342.

The sealing liquid L2 may contain a surfactant. Examples of surfactants include an ionic surfactant, a nonionic surfactant, and an amphoteric surfactant.

The inclusion of a surfactant in the sealing liquid is expected to prevent the enzyme contained in the wells 341 from being inactivated due to direct contact with the oil of the sealing liquid L2. It is also expected to prevent the target and reagent components required for reactions from dissolving into the oil.

In addition, if a reaction reagent such as ALP or FDP is added to the wells 341 in advance, and then the wells 341 are sealed with the sealing liquid L2 before adding the target bound to particles such as beads to the flow channel 330, the inclusion of a surfactant in the sealing liquid L2 is expected to facilitate the introduction of the particles into the wells 341 even in the presence of oil.

It is also preferable to dissolve a lipid in the sample solution L, and deliver a liquid containing a lipid again after delivering the sealing liquid L2 to the flow channel 330.

This allows a lipid bilayer membrane to be formed at the opening of each well 341, and the wells 341 can be individually sealed with the lipid bilayer membranes to form the sealed wells 342.

Examples of the lipid to form the lipid bilayer membrane include 1,2-dioleoyl-sn-glycero-3-phosphoethanolamine (DOPE), 1,2-dioleoyl-sn-glycero-3-phosphoglycerol (DOPG), and mixtures thereof, but are not limited thereto.

Next, the above-described <<Target Molecule Detection>> and <<Fluorescence Observation> > are carried out.

According to this method, it is possible to suppress false positives and improve the accuracy of target molecule detection.

### Examples

The present invention will be described in more detail referring to the examples described below, but the present invention is not limited to the examples.

### <Experimental Example 1>

### (Production of Fluidic Device)

First, a test fluidic device corresponding to one first well W1 in the fluidic device shown in Fig. 9 was produced by injection molding. A cycloolefin polymer was used as the material of the well plate. A cover member 12 made of a cycloolefin polymer colored by adding carbon black was produced by injection molding, and bonded by laser welding to produce the fluidic device.

The first well W1 had a depth (the distance from the upper surface of the well plate to the top of the wall member) of 30 µm.

The second wells W2 had an opening diameter (major diameter) of 10 µm a depth of 15 µm. The volume Vd of each second well W2 was 1100 fL. The total volume of the second wells was approximately 20% of the overall volume of the fluidic device.

### (Model Sample)

In this example, magnetic beads (hereinafter, magnetic beads) were used as a cell model. In addition, as a model simulating cytokines secreted from cells, DNA was bound to the magnetic beads, and the DNA was hybridized by allowing it to pair with its complementary DNA strand.

### (Sample Solution)

As the sample solution, a solution containing the following substances were prepared using water (AccuGENE Molecular Biology Grade Water (manufactured by LONZA)).
Detection oligo DNA: 0.25 µmol/L Allele probe (Fasmac Co., Ltd.)
Detection oligo DNA: 5 nmol/L Invader probe (Fasmac Co., Ltd.)
Fluorescent substrate: 2 µmol/L 1-1 Alexa 488 (Japan Oligo Service)
Buffer substance: 50 mmol/L Tris-HCl (manufactured by NIPPON GENE CO., LTD., pH: 8.5)
Salt: 20 mmol/L MgCl₂ (manufactured by SIGMA-ALDRICH)
Surfactant: 0.05 mass% Tween 20 (manufactured by SIGMA-ALDRICH)
Detection enzyme: 0.05 mg/ml Fen1
Simulated cell: Magnetic beads having capture DNA bound thereto and hybridized with complementary DNA (target DNA)
Magnetic beads: Magnosphere MS300/Carboxyl (manufactured by JSR Corporation), having a diameter of 3 µm

As the simulated cell, a model sample was obtained by binding DNA for capturing DNA (Fasmac) to the above-described magnetic beads whose surface was modified with carboxyl groups, and hybridizing it with the target DNA (Fasmac).

### (Sealing Liquid)

An oil (Fluorinert FC-40, manufactured by SIGMA-ALDRICH) was used as the sealing liquid.

### (Microscopic Observation)

The following device was used.
Microscope: BZ-800 (manufactured by KEYENCE CORPORATION)
Objective lens: CFI Plan Apochromat Lambda 10X (manufactured by Nikon Corporation)
Filter 1: BZ-X filter Texas Red (manufactured by KEYENCE CORPORATION)
Filter 2: BZ-X filter GFP (manufactured by KEYENCE CORPORATION)

### [Examples]

After MS300/Carboxyl was thoroughly stirred using a Vortex mixer, 10 µL of the mixture was extracted and added to a 1.5 ml tube.

Using a magnetic stand, the beads were washed once with 200 µL of reaction solution (buffer: 0.1 mol/L MES buffer, pH 5.0, pH adjusted with a NaOH solution). Then, 920 µL of reaction buffer was added.

After adding 80 µL of a 100 µmol/L target DNA capture DNA solution diluted with pure water, the mixture was stirred for 30 minutes.

Next, EDC that had been diluted with a reaction buffer to 10 mg/mL was added, and the mixture was stirred with a rotator for 3 hours.

Using a magnetic stand, the beads were washed with 1 ml of PBS-T (repeated three times).

After adding 200 µL of PBS-T (a PBS solution containing 0.05% Tween 20) and storing the mixture at 4°C, the target DNA was diluted to 1 nmol/L with a 0.1 ng/µL carrier DNA solution.

46 µL of PBS-T, 2 µL of the 1 nmol/L target DNA solution, and 2 µL (6 × 104 beads) of capture DNA-bound bead solution were weighed and placed in a 1.5 ml tube. The mixture was stirred in the vortex mixer for 15 minutes and then washed three times with 100 µL of PBS-T. 200 µL of 0.05% Tween 20 solution was added.

### (Introduction of Mimetic Cell)

The bead solution was diluted with the 0.05% Tween 20 solution so as to contain one bead, and the ICA reagent was mixed. 8 µL of the mixture of the bead solution and ICA reagent was added to the test fluidic device. It was confirmed under a microscope that only one bead is present in the test fluidic device.

### (Mimetic Cytokine Release)

The fluidic device containing the above-described mixture was heated at 50°C for 30 seconds using a heater.

### (Mimetic Cytokine Detection)

500 µL of FC40 (sealing liquid) was injected into the fluidic device using a syringe having a plastic needle. The fluidic device was then heated at 66°C for 7 minutes using a heater.

The number of wells with a fluorescence intensity of 1000 or more was counted using a BZ analyzer (KEYENCE CORPORATION).

### [Comparative Example (Blank Test)]

The test was carried out in the same manner as in Example 1, except that no target DNA was bound to the beads. It was confirmed under a microscope that only one bead is present in the test fluidic device.

As a result of the evaluation, in the example, the number of second wells in which fluorescent light was observed was 49 out of a total of 900,000 second wells. On the other hand, in the comparative example, the number of second wells in which fluorescent light was observed was 24 out of a total of 900,000 second wells. In the example, the number of wells in which DNA was detected was 25 (49 - 24).

Since the total volume of the second wells is approximately 20% of the total input solution, combined with the amount of DNA that was contained in the part of the sample solution replaced with sealing liquid, it can be estimated that 125 strands of DNA were bound to one bead.

The above results confirmed the usefulness of the present invention.

### <Experimental Example 2>

In the present examples and comparative example, a liquid containing FDP was placed inside the substrate 310 of the fluidic device 300 shown in Fig. 12 and a large number of wells 341 formed in the upper surface of the substrate 310. After sealing the wells with oil, the liquid was left to stand for a certain period of time, and the number of nonspecific reactions was counted by observing with a fluorescence microscope.

### (Production of Fluidic Device)

First, a fluidic device having the structure shown in Fig. 12 was produced. A substrate 310 made of a cycloolefin polymer and having the well array 340, formed by injection molding, and a cover member 320 made of a cycloolefin polymer colored by adding carbon black were bonded by laser welding to produce the fluidic device. A double-sided tape served as the protrusion part 321, and the flow channel 330 had a height (the distance between the upper surface 311 of the substrate 310 and the surface of the cover member 320 facing the substrate 310) of 30 µm. The cover member 320 was provided with the introduction port 322 and the discharge port 323. The wells 341 of the well array 340 had a diameter of 10 µm and a depth of 15 µm. The volume Vd of each well 341 was 1100 fL.

### (Introduction of Pure Water)

200 µL of pure water was added to the fluidic device to fill the well array 340 with pure water. AccuGENE Molecular Biology Grade Water (manufactured by LONZA) was used as the pure water.

Then, 20 µL of FDP solution was added to the fluidic device to fill the well array 340 with the FDP reagent. The composition of the FDP solution was as follows: pure water (AccuGENE Molecular Biology Grade Water (manufactured by LONZA)), one of five systems of FDP (model number: 11600, manufactured by AAT Bioquest), namely, 50, 100, 200, 400, and 800 µM, 1 M DEA (pH 9.5) (model number: 099-03215, manufactured by WAKO)), 1 mM MgCl₂ (model number: M1028-100ML, manufactured by SIGMA), and 0.05% [v/v] tween 20 (model number: P7949-100ML, manufactured by SIGMA). The system with an FDP concentration of 50 µM was used for the comparative example, and the systems with FDP concentrations of 100, 200, 400, and 800 µM were used for the examples.

### (Sealing of Wells)

500 µL of oil was added to the fluidic device 300 to seal the wells 341. An oil (Fluorinert FC-40, manufactured by SIGMA-ALDRICH) was used as the sealing liquid.

### (Reaction)

The mixture was left to stand for 40 minutes at room temperature. That is, in the present examples and the comparative example, the fluidic device 300 was left to without ALP, which is an enzyme for which FDP serves as a substrate.

### (Microscopic Observation)

After the above reaction time had elapsed, each system was observed under a fluorescence microscope, and fluorescent images were obtained. The following device was used.

Microscope: BZ-800 (manufactured by KEYENCE CORPORATION)
Objective lens: CFI Plan Apochromat Lambda 10X (manufactured by Nikon Corporation)
Filter: BZ-X filter GFP (manufactured by KEYENCE CORPORATION)
Excitation light: 100%
Exposure time: FDP (50 µM): 1 s
   FDP (100 µM): 1/1.5 s
   FDP (200 µM): 1/3 s
   FDP (400 µM): 1/3.5 s
   FDP (800 µM): 1/5 s

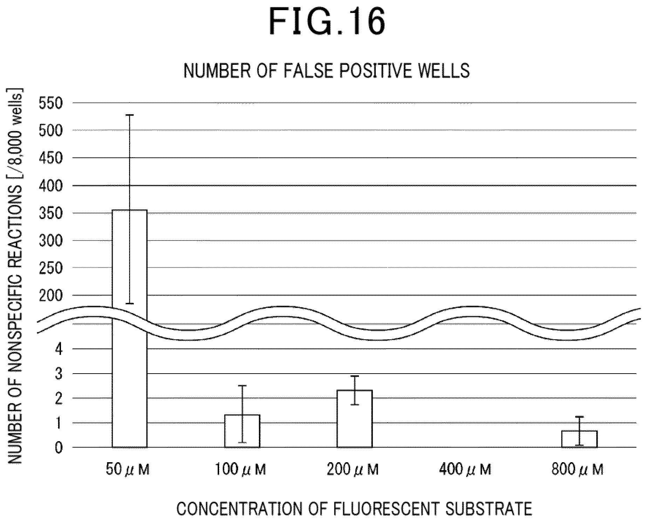

Based on the images obtained by fluorescence observation for each substrate concentration, wells with a fluorescence intensity higher than the fluorescence intensity at +3σ relative to the average of 8,000 wells were counted as positive wells (i.e., false positive wells) for each FDP concentration. Table 1 shows the data on the number of wells in which false positive reactions were observed for each substrate concentration, and Fig. 16 shows a graph of this data set.

**[Table 1]**

| | Substrate concentration | False positive reaction | | | AVERAGE | Standard deviation |
|---|---|---|---|---|---|---|
| Comp. Ex. | 50 µM | 430 | 477 | 159 | 355.33 | 171.64 |
| Ex. 1 | 100 µM | 2 | 2 | 0 | 1.33 | 1.15 |
| Ex. 2 | 200 µM | 2 | 3 | 2 | 2.33 | 0.58 |
| Ex. 3 | 400 µM | 0 | 0 | 0 | 0 | 0 |
| Ex. 4 | 800 µM | 1 | 1 | 0 | 0.67 | 0.58 |

As can be seen from Table 1 and Fig. 16, the counting results showed that, in the case of the 50 µM FDP substrate concentration, an average of 355.33 false positive wells were observed out of 8,000 wells (n = 3). On the other hand, the samples with concentrations of 100 µM to 800 µM only had an average of 0 to 2.33 false positive reactions. These false positive reactions are considered to be caused by self-decomposition of FDP due to the excitation light of the fluorescence microscope.

The above results indicate that it was possible to effectively suppress false positive reactions with the four systems having FDP concentrations of 100 µM, 200 µM, 400 µM, and 800 µM in the reaction solution contained in the wells 141.

In addition, it was found that the system with an FDP concentration of 400 µM in the reaction solution was most effective in suppressing false positive reactions.

Thus, it was found that, by setting the FDP concentration within the above range, the occurrence of false positives can be effectively suppressed, thereby significantly improving the accuracy of target measurement based on the decomposition reaction of FDP by alkaline phosphatase.

Note that, if ALP is added to the wells 341 to cause an enzymatic reaction, the above suitable FDP concentration range refers to the suitable FDP concentration range in the reaction solution in the wells 341 to which ALP, FDP, the target, and the like have been added. This FDP concentration refers to the concentration of FDP at the time the detection reaction (i.e., enzymatic reaction) using ALP and FDP has started, after the target, ALP, and FDP have been added to the wells.

Further, the above concentration range suitable for suppressing false positives is not limited to FDP, but can also be applied to a concentration range of another substrate such as pNPP or MFP when such substrate is used as the substrate for ALP in digital measurement. When MFP is used, similarly to ALP, wells 341 in which a fluorescence reaction is observed can be counted as positive. On the other hand, when pNPP is used, wells 341 in which the liquid turned yellow can be counted as positive.

### (Discussion of Experimental Results)

When the FDP concentration is lower than 100 µM, the exposure time to the excitation light needs to be increased, and the substrate is more likely to self-decompose due to the excitation light. Further, since the concentration of FDP is low, even a slight decomposition may result in a high rate of increase in fluorescence intensity, which facilitates the occurrence of false positives. On the other hand, when the FDP concentration is 100 µM or more, there is a sufficient amount of substrate to reduce the exposure time. In addition, even if there is some decomposition, since the proportion of decomposed FDP relative to the FDP concentration in the solution is low, false positive reactions are less likely to be detected.

When FDP with a concentration higher than 800 µM is added for the enzymatic reaction, not only it is possible that false positive reactions increase, it may not be suitable for use in terms of solution preparation and cost.

### [Industrial Applicability]

According to the present invention, a fluidic device capable of detecting target molecules released from the target substance in a solution, with high sensitivity, can be provided. A method for detecting target molecules using such fluidic device can also be provided.

### [Reference Signs List]

11, 21 ... Well plate (substrate)
11a, 12a, 21a, 22a ... Upper surface
12, 22 ... Cover member
13, 23 ... Wall member
13a ... Top
100, 200, 300, 400 ... Fluidic device
121, 221 ... Inlet
122, 222 ... Outlet
FC, 330 ... Flow channel
L ... Sample solution
L1 ... Detection reagent
L2 ... Sealing liquid
M1 ... Target substance
M2 ... Target molecule
W1 ... First well
W2 ... Second well

## Claims

1. A fluidic device comprising:
a substrate;
a wall member provided on the substrate; and
a cover member facing the substrate and in contact with the wall member, wherein
the cover member has an inlet and an outlet that pass through the cover member in a thickness direction,
a flow channel through which a fluid flows is formed between a top of the wall member and the cover member,
the fluidic device comprises a plurality of first wells surrounded by the substrate and the wall member, and
the fluidic device comprises a plurality of second wells provided in an upper surface of the substrate corresponding to bottoms of the first wells.

2. The fluidic device according to claim 1, wherein the cover member is detachable from the wall member.

3. The fluidic device according to claim 1 or 2, wherein an opening of the first wells has a major diameter of 1 µm or more and 1 cm or less.

4. The fluidic device according to claim 1, wherein the first wells have an aspect ratio of 0.002 or more and 3 or less.

5. The fluidic device according to claim 1, wherein an opening of the second wells has a major diameter of 1 nm or more and 1 mm or less.

6. The fluidic device according to claim 1, wherein the second wells have an aspect ratio of 0.05 or more and 3 or less.

7. A method for detecting a target molecule using the fluidic device according to claim 1, the method including:
filling the first wells independently with a sample solution containing a target substance;
releasing the target molecule from the target substance in, among the first wells, first wells in which one unit of the target substance is placed;
distributing the target molecule to the second wells independently so that one molecule is placed in one second well; and
causing a reaction between the target molecule and a detection reagent to detect the target molecule.

8. The method for detecting a target molecule according to claim 7, wherein, in the filling step, a sealing liquid is introduced into the fluidic device to seal the sample solution in the first wells by the sealing liquid.

9. The method for detecting a target molecule according to claim 8, wherein the sealing liquid is fluorinated oil.

10. The method for detecting a target molecule according to claim 7, wherein the sample solution contains a surfactant.

11. The method for detecting a target molecule according to claim 7, the method further including, prior to the filling step, obtaining the sample solution by adjusting a concentration so that one unit of the target substance is placed per first well based on a volume of the fluidic device.

12. A method for detecting a target molecule using a fluidic device having a well, the method including:
placing a reaction solution in the well for reaction between the target molecule, alkaline phosphatase immobilized on a binder that binds to the target molecule, and a substrate that changes visually when dephosphorylated by the alkaline phosphatase; and
visualizing and detecting a presence of the target molecule contained in the well by using a dephosphorylation reaction of the substrate by the alkaline phosphatase in the well, wherein
at a start of the step of visualizing and detecting the presence of the target molecule, a concentration of the substrate in the reaction solution contained in the well is 80 µmol/L or more and 800 µmol/L or less.

13. The method for detecting a target molecule according to claim 12, wherein the reaction solution contains a surfactant.

14. The method for detecting a target molecule according to claim 12 or 13, further including sealing the well containing the reaction solution with oil.

15. The method for detecting a target molecule according to claim 12 or 13, wherein the binder is an antibody.

16. The method for detecting a target molecule according to claim 12 or 13, wherein, in the step of placing the target molecule in the well, the target molecule is retained in the well using particles to which a capture means for capturing the target molecule is immobilized.

17. The method for detecting a target molecule according to claim 12 or 13, wherein the reaction solution contains a buffer solution.
